# EUROPEAN PATENT SPECIFICATION

(11) **EP 0 804 929 B1**
(45) Date of publication and mention of the grant of the patent: **18.12.2002**
(21) Application number: 97303038.0
(22) Date of filing: 02.05.1997
(51) Int. Cl.: A61K 35/78, A61P 33/12

(54) **Treatment of schistosomiasis**
Behandlung von Schistosomiasis
Traitement de la schistosomose

(30) Priority: 02.05.1996 EG 38096
(43) Date of publication of application: 05.11.1997
(73) Proprietor: Pharco Pharmaceuticals, Bolkly, Alexandria (EG)
(72) Inventor: Massoud, Ahmed Mohamed Ali, Dr., Cairo (EG)
(74) Representative: Baldock, Sharon Claire

(56) References cited:
- EP-A- 0 513 671
- WO-A-96/05849
- BE-A- 751 806
- MASSOUD AM ET AL.: "EXPERIMENTAL STUDIES DEMONSTRATING THE ANTISCHISTOSOMAL ACTIVITY OF MYRRH, COMMIPHORA MOLMOL" THE AMERICAN JOURNAL OF TROPICAL MEDICINE AND HYGIENE, vol. 55, no. 2 SUPPL., August 1996, pages 233-234, XP002050376

## Description

The present invention provides means for the treatment of schistosomiasis and in particular the use of an extract from Commiphora molmol, such as for example, oleo-gum-resin (Myrrh) or a derivative thereof, for preparing a medicament for the treatment of schistosomiasis, and pharmaceutical compositions containing several extracts from Commiphora molmol.

Schistosomiasis is increasing in incidence despite concerted efforts to control the disease in all the endemic areas. While a multiprolonged method of control using health education, sanitation and snail control has been used, chemotherapy and chemoprophylaxis play the most important and crucial role in containing/preventing the transmission of the disease.

Praziquantel is currently the drug of choice for the treatment of any kind of schistosomiasis. The only limitation is the cost which restricts its use in many developing countries.

While effective, safe drugs for mass chemotherapy are being developed, the problem of therapeutic failure and drug resistance is being reported from certain developing countries. Under these circumstances, alternative drugs must be resorted to *(Drugs, 42(3) : 379-405).*

Mass treatment, a crucial goal in the eventual control of schistosomiasis, awaits a well-tolerated and nontoxic drug that will ultimately prove to be effective where cure is definite.

Myrrh is an oleo-gum-resin obtained from the stem of Commiphora molmol (Family *Burseraceae*) growing in north-east Africa and Arabia. The drug is chiefly collected in Somali land. Much of the secretion from the plant is obtained by spontaneous exudation from the cracks and fissures which commonly form in the bark, and some maybe obtained from incisions. The yellow-ish white, viscous fluid soon hardens in the heat to a reddish-brown mass. Myrrh contains approximately, 7-17% of volatile oil, 25-40% of resin, 57-61% of gum and some 3-4% of impurities.

Plant mixture extract may prove to be a useful therapeutic agent in the treatment of Non-Insulin Dependent Diabetes Mellitus (NIDDM) ((*1991*): *Diabetes Res. 18(4):163-8*). Commiphora molmol (Myrrh) activity was found to be equivalent to that of the standard cytotoxic drug cyclophosphamide ((1993): *Cancer Chemother. Pharmacol., 33(2):130-8*). Treatment with Commiphora molmol (Myrrh) (250 and 500 mg/kg/day) was found to be cytotoxic in Ehrlich solid tumors cells. The antitumor potential of C.molmol was comparable to the standard cytotoxic drug cyclophosphamide. The present study confirmed the cytotoxic and anticarcinogenic potential of C.molmol ((1994) : *Chemotherapy, 40(5):337-47)*. T-Cadinol; a sesquiterpene active constituent of scented myrrh was shown to have a concentration - dependent smooth muscle relaxing effect on the isolated guinea pig ileum ((1991): *Planta. Med., 57(4):352-6)*. The resin (Myrrh) is widely used in Somalia to treat stomach complaints and diarrhoea ((1991): *Phytotherapy Research, 5(3):142-4*). Sesquiterpene T-Cadinol caused bacterial lysis and subsequent fatal loss of intracellular components in Staphylococcus aureus ((1992): *Phytotherapy Research, 6(2):94-8*). Myrrh, natural gum resins have been used to relieve pain and swelling due to traumatic injury by patching test ((1993): *Contact Dermatitis, 28(2)@89-90*). Myrrh has found recent pharmalogical application in the reduction of cholesterol and triglycerides ((1991): *J.* used for therapy of aphthous ulcers (stomatitis ulcer) (*AM. Drug., 202:90*).

Myrrh is approved by the FDA for food use (21 CFR 172.510), and was given Generally Recognised As Safe (GRAS) status as a flavour ingredient (No. 2765) by the Flavour Extract Manufacturer's Association (FEMA). The Council of Europe (1981) included myrrh in the list of plants and parts thereof, which are acceptable for use in foods (*Foods Chem. Technol., 1992*).

Accordingly, there is provided by a first aspect of the present invention the use of an extract from the Commiphora molmol plant in the preparation of a medicament for the treatment of schistosomiasis.

Preferably, the extract comprises, additionally or alternatively, a volatile oil, resin or gum extracted from the plant Commiphora molmol and even more preferably oleo-gum resin (Myrrh).

According to a further aspect of the present invention there is also provided a pharmaceutical composition comprising a pharmaceutically effective amount of an extract of Commiphora molmol comprising additionally or alternatively a volatile oil, resin or gum other than the oleo-gum resin, together with a pharmaceutically acceptable carrier diluent or excipient therefor. In one embodiment the pharmaceutical composition is provided in, or in the form of a gelatin capsule.

Methods for extracting volatile oils from Commiphora molmol include the steps of;
(a) covering ground Commiphora molmol with a layer of water;
(b) passing steam through the resulting mixture;
(c) condensing volatile oil in a condensing chamber;
(d) separating the oil layer from the aqueous layer.

Further, volatile oils may be extracted from Commiphora molmol which method comprises;
(a) petroleum ether extraction;
(b) evaporating the petroleum ether to give the oil fraction.

A method of extracting resins from Commiphora molmol after separation of volatile oils as described herein comprises;
(a) exhausting the powdered drug with alcohol after separation of the volatile oil component;
(b) evaporating alcohol extract;
(c) collecting the precipitated resin, washing and drying.

Resins can also be extracted after separating the volatile oil as described herein, which method comprises;
(a) petroleum ether extraction;
(b) extracting ether with methanol;
(c) evaporating the methanol to give resin fraction.

The invention may be more clearly understood by the following description of an exemplary embodiment thereof, which is given by way of example only and with reference to the accompanying figures wherein;

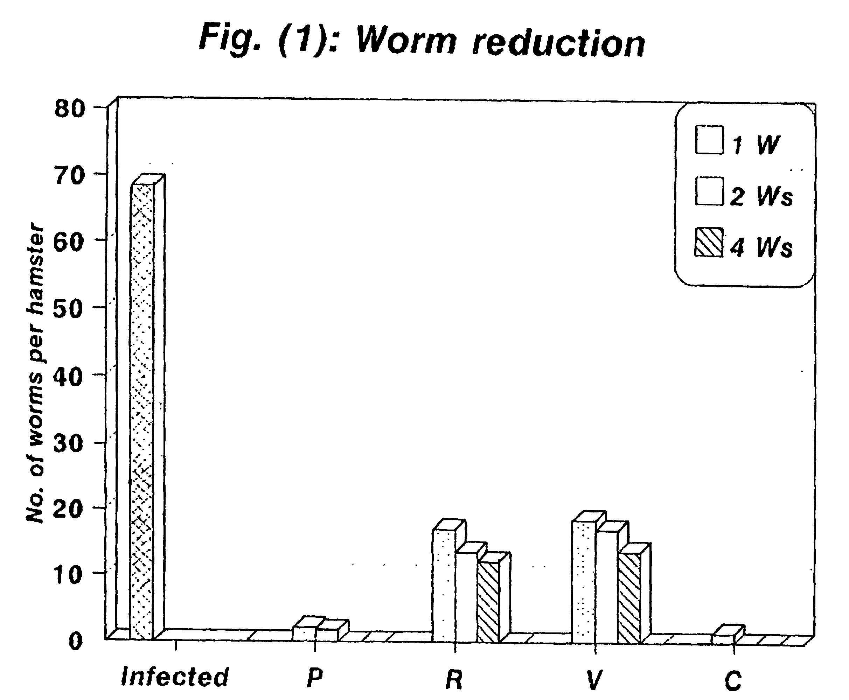

- Figure 1: is a graphic representation of the worm reduction in a hamster following treatment with the combination therapy (Table 1).

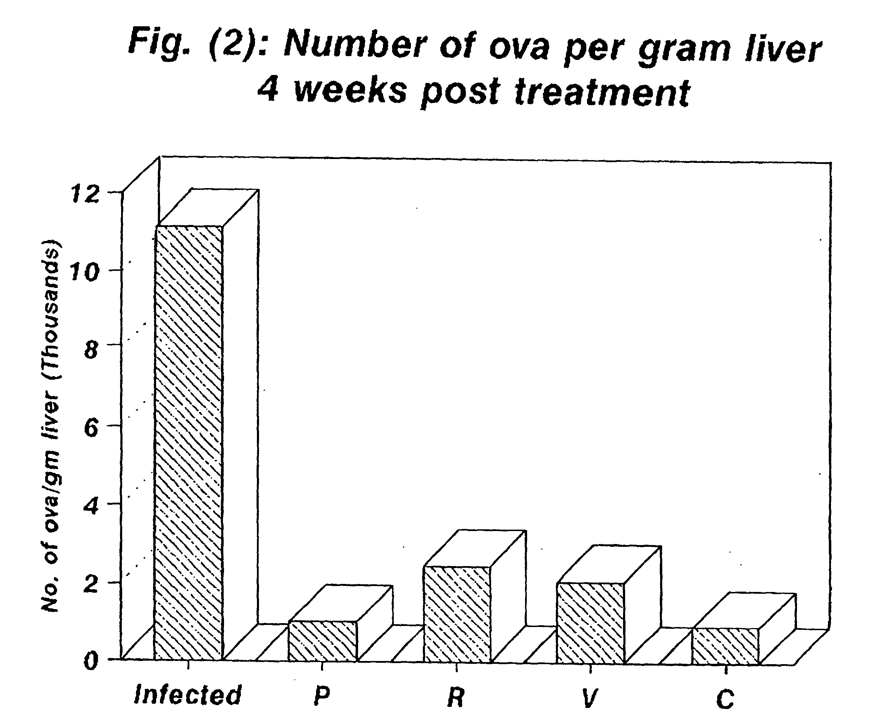

- Figure 2: is a graphic representation of the number of ova per gram liver, 4 weeks post treatment.

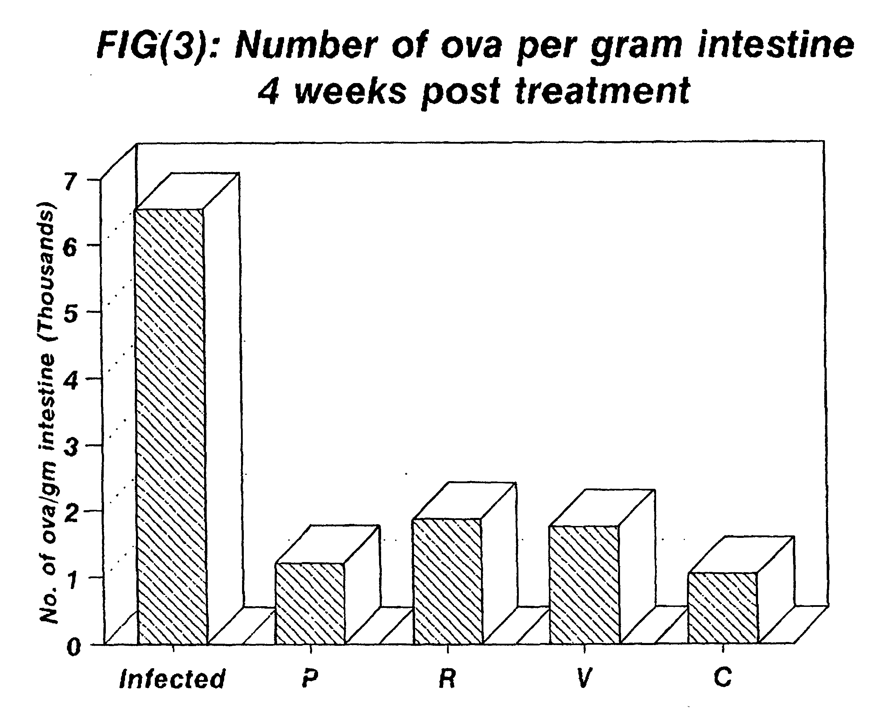

- Figure 3: is a graphic representation of the number of ova per gram intestine 4 weeks post treatment.

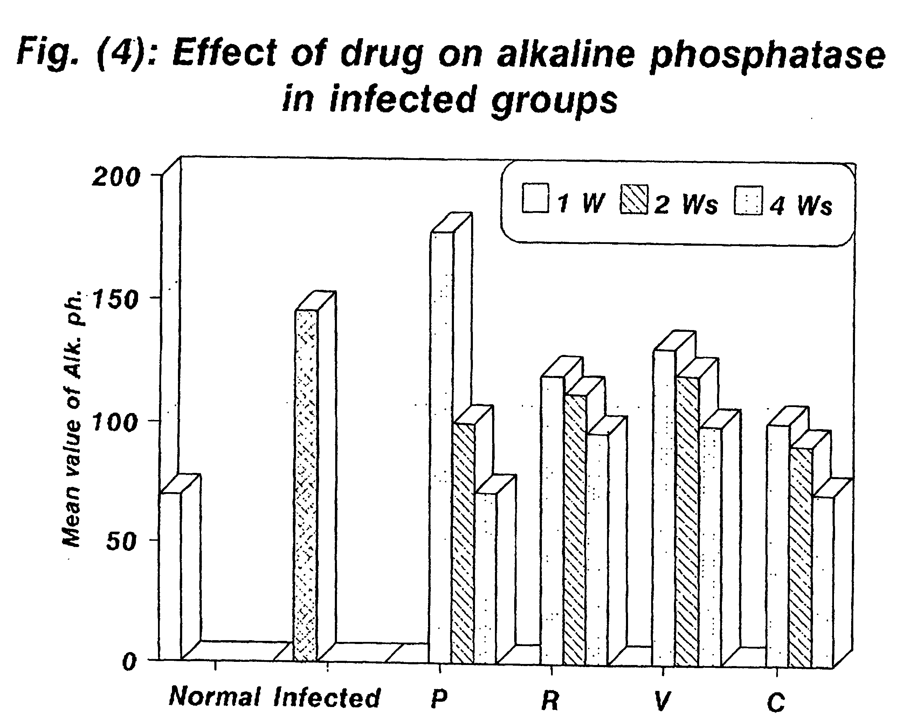

- Figure 4: is a graphic representation of the effect of the drug on alkaline phosphatase in infected groups.

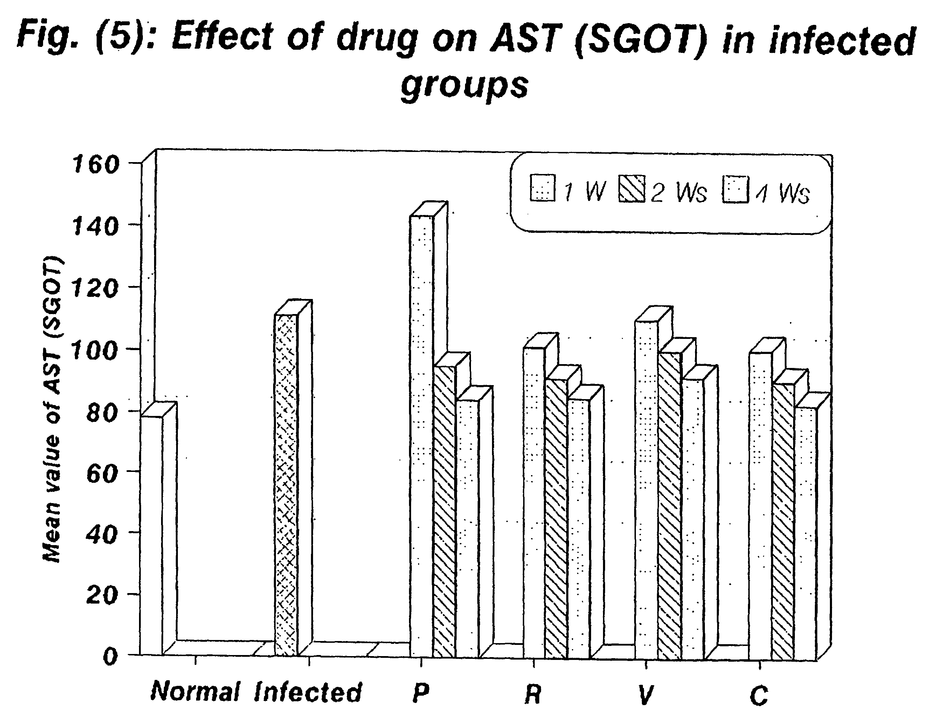

- Figure 5: is a graphic representation of the effect of the drug on (AST) Aspartate Aminotransferase, Serum Glutamic Oxalacetic Transaminase (SGOT) in infected groups.

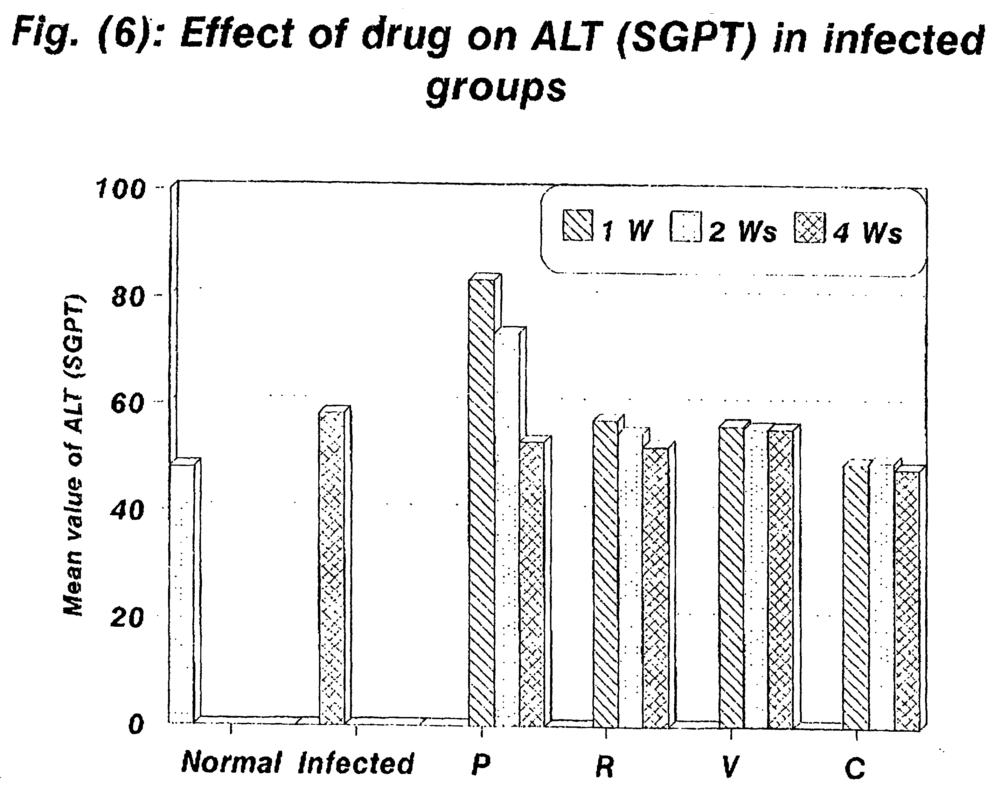

- Figure 6: is a graphic representation of effect of the drug on (ALT) Alanine Aminotransferase, Serum Glutamic Pyruvic Transaminase (SGPT) in infected groups.

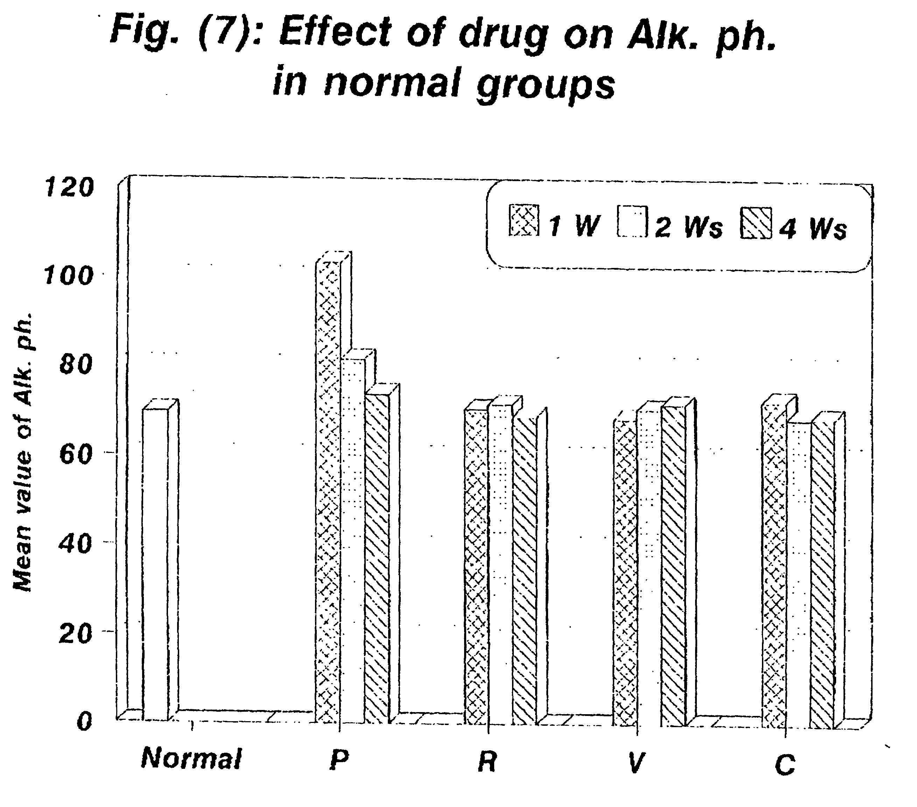

- Figure 7: is a graphic representation of the effect of the drug on alkaline phosphatase in normal groups.

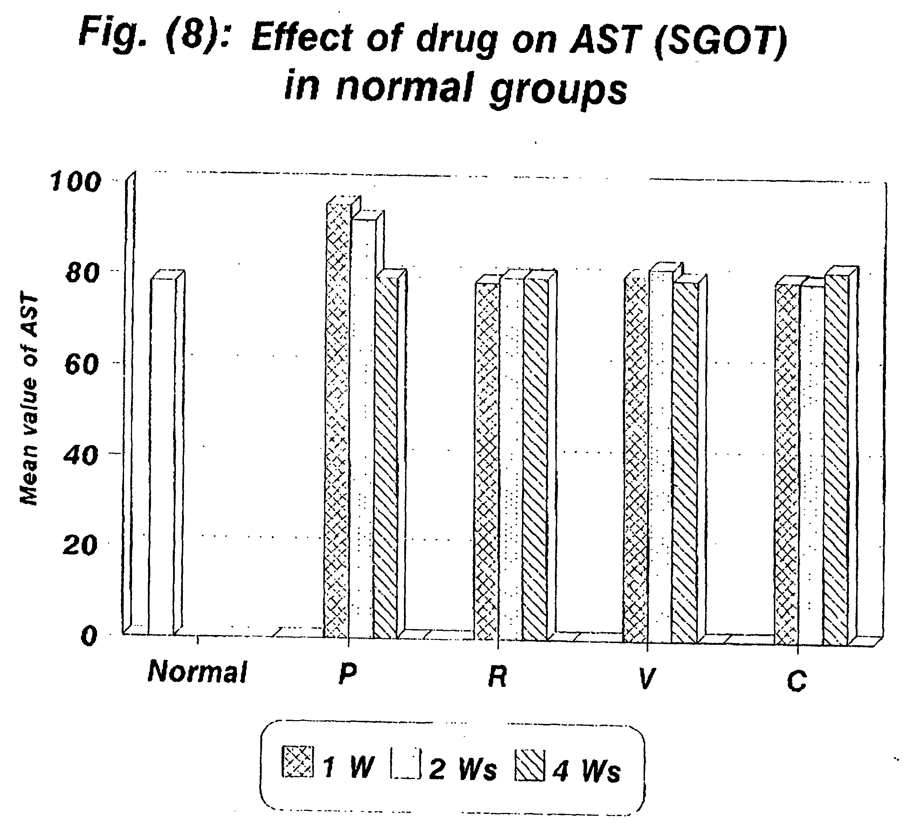

- Figure 8: is a graphic representation of the effect of the drug on AST (SGOT) in normal groups.

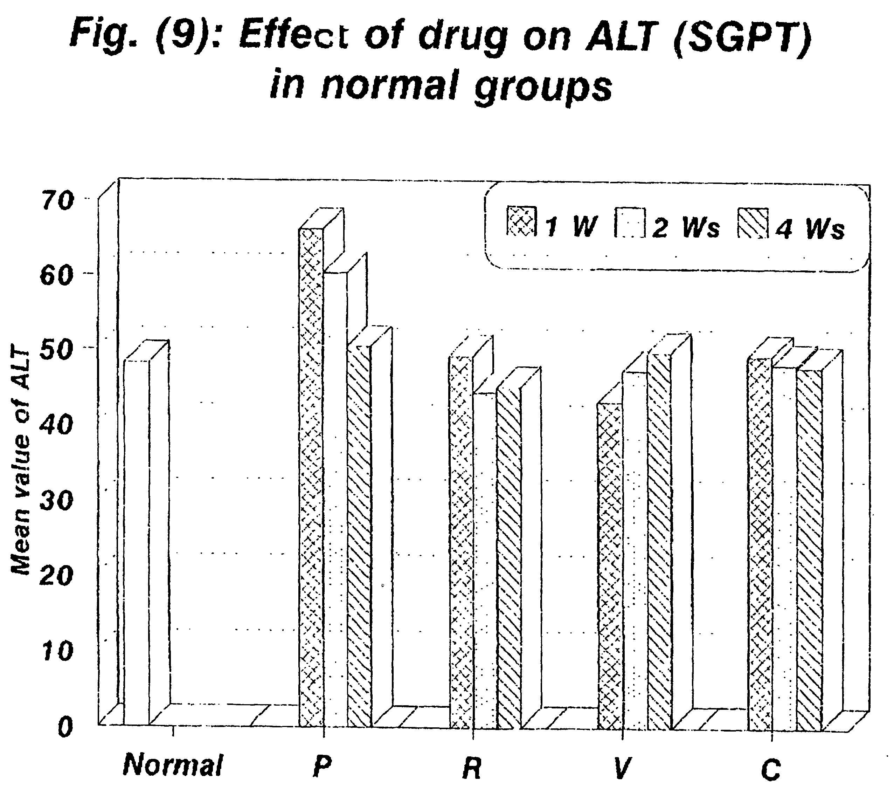

- Figure 9: is a graphic representation of the effect of drug on ALT (SGPT) in normal groups.

### Constituents of the crude plant mainly included:

1. Volatile oils, containing heerabolene, cadinene, elemol, eugenol, cuminaldehyde, numerous furanosesquiterpenes including furanodiene, furanodienone, curzerenone, lindestrene, 2-methoxyfuranodiene and other derivatives.
2. Resins, including [[alpha]]-, [[beta]]-and[[gamma]]-commiphoric acids, commiphorinic acid, heeraboresene, [[alpha]]-and[[beta]]-heerabomyrrhols and commiferin.
3. Gums, composed of arabinose, galactose, xylose and 4-0-methylglucuronic acid.

### Methods of Preparation

1. Volatile Oils
   (a) water and steam distillation: the plant is ground and immediately covered with a layer of water and steam is passed through the mixture using pipes. The volatile oil is condensed in the condensing chamber. The oil layers are separated from the aqueous layer;
   (b) petroleum ether extraction, 1 kg of Myrrh powder is extracted with 3 Ls petroleum ether 3 times; 1 L. each time. Evaporate the petroleum ether to get the oil fraction.
2. Resins
   (a) alcohol extraction: the powdered drug after separation of volatile oil component is exhausted with ethanol. Alcohol extract is evaporated and the precipitated resin is collected, washed and dried;
   (b) petroleum ether extraction: the powdered drug after extraction with petroleum ether is extracted with methanol 3 times; 200 ml each time. The methanol is then extracted to get the resin fraction.

### Experimentation:

### Material and Methods:

In our study five groups of hamsters (30 each) were experimentally infected with 120 ± 10 cercariae of an Egyptian strain of S. *mansoni (1965: Am. J*. *Trop. Med. Hyg., 14(4):579-80*). The weight of animals used in this study ranged from 80 to 120 gm and their age ranged from 8 to 10 weeks.

One group was treated by the crude plant emulsion "Myrrh", 180 mg/kg. A second group was treated with the resin active gradient separated from the plant 60 mg/kg. The third group was treated with the volatile oil active gradient separated from the plant 30 mg/kg. A fourth group was treated with a combination of resin and volatile oil (60 mg/kg and 30 mg/kg respectively). Treatment started in all groups eight weeks post infection. The fifth group served as an infected non-treated control group. In all treated groups, the drug preparations were given in an emulsion with distilled water on three equally divided daily oral doses.

Five other groups of non-infected normal hamsters (30 each) were tested; four groups were treated with the same drug regimens as in the infected groups and the fifth one served as normal non-infected non-treated control group.

Hamsters were sacrificed by decapitation after fasting overnight for one week, two weeks and four weeks respectively after the end of treatment: (10 animals each time in each group). Blood was collected, centrifuged for separation of serum for estimation of enzymes: alkaline phosphatase, ALT(SGPT) and AST(SGOT).

The therapeutic efficacy of the drugs was tested parasitologically by studying:
1. Worm Load: immediately after decapitation, the worms from infected animals were collected counted from the portal and mesenteric veins as well as from the liver by perfusion *(1956): Rev. Bras. Molar.,* *8:589-97*).
2. Oogram Studies: including microscopic examination of press preparations from intestinal fragments of infected animals (*1965*) *Am. J. Trop. Med. Hyg., 14(3):363-9*. Three fragments of whole intestine wall (small intestine) each measuring approximately 1 cm in length were cut off. One hundred eggs were counted per each fragment and classified according to the four different stages of their development *(1962) Am. J. Trop. Med. Hyg., 11(2):201-25.*
   Eggs of the first stage have small embryos occupying approximately one third of the diameter of the eggs. Embryos of the second stage are slightly larger than half the transverse diameter. Eggs of the third stage show an embryo whose size corresponds to approximately two-thirds the diameter of the eggs. Eggs of the fourth stage show embryos that occupy almost the whole of the egg shell. The mature eggs contain a fully developed miracidium with signs of miracidia vitality (activity of flame cells and beating of cilia).
   Dead eggs appeared semitransparent, granular, darkened with retracted embryos. The mean percentage of each development stage of eggs in the three fragments was calculated in relation to 300 eggs.
   A dose of any schistosomicidal drug is considered to be effective against *S.mansoni* worms when the oogram showed 50% or more mature eggs and absence of one or more stages of immature eggs (*1962*) *Am. J. Trop. Med. Hyg., 11(2):201-25*, they stated that active schistosomicidal drugs are able to produce and increase in the percentage of mature eggs in relation to viable eggs up to 100% after variable periods.
3. Egg count in liver and intestine: ova of homogenous emulsions were counted after being spread on slides and number of ova per gram tissues were calculated (*1965*) *Am. J. Trop. Med. Hyg., 14(3):363-9.*

### Results

The drug has resulted in a marked reduction in worm load reaching 100% two weeks after treatment in a group which received the combination therapy (Table: 1 & Fig: 1). Furthermore, the drug caused effective oogram changes with more antischistosomal activity in the groups which received the combination therapy and the crude plant (Table:2). There was significant reduction in the number of ova in the liver and intestine which was more evident in groups which received the combination therapy and the crude plant and the maximal reduction was reached after one month (Table: 3 & Fig: 2, 3). The estimated serum enzymes showed a transient rise which was noticed one week post treatment only in those groups that received the crude plant (infected treated group and non-infected normal treated group) and levels were normalized one week later (Table: 4 & Fig: 4, 5, 6). On the other hand, resin, volatile oil and combination therapy did not cause any significant changes in enzyme levels in all normal non-infected groups all through the period of the experiment (Table: 5 & Fig: 7, 8, 9).

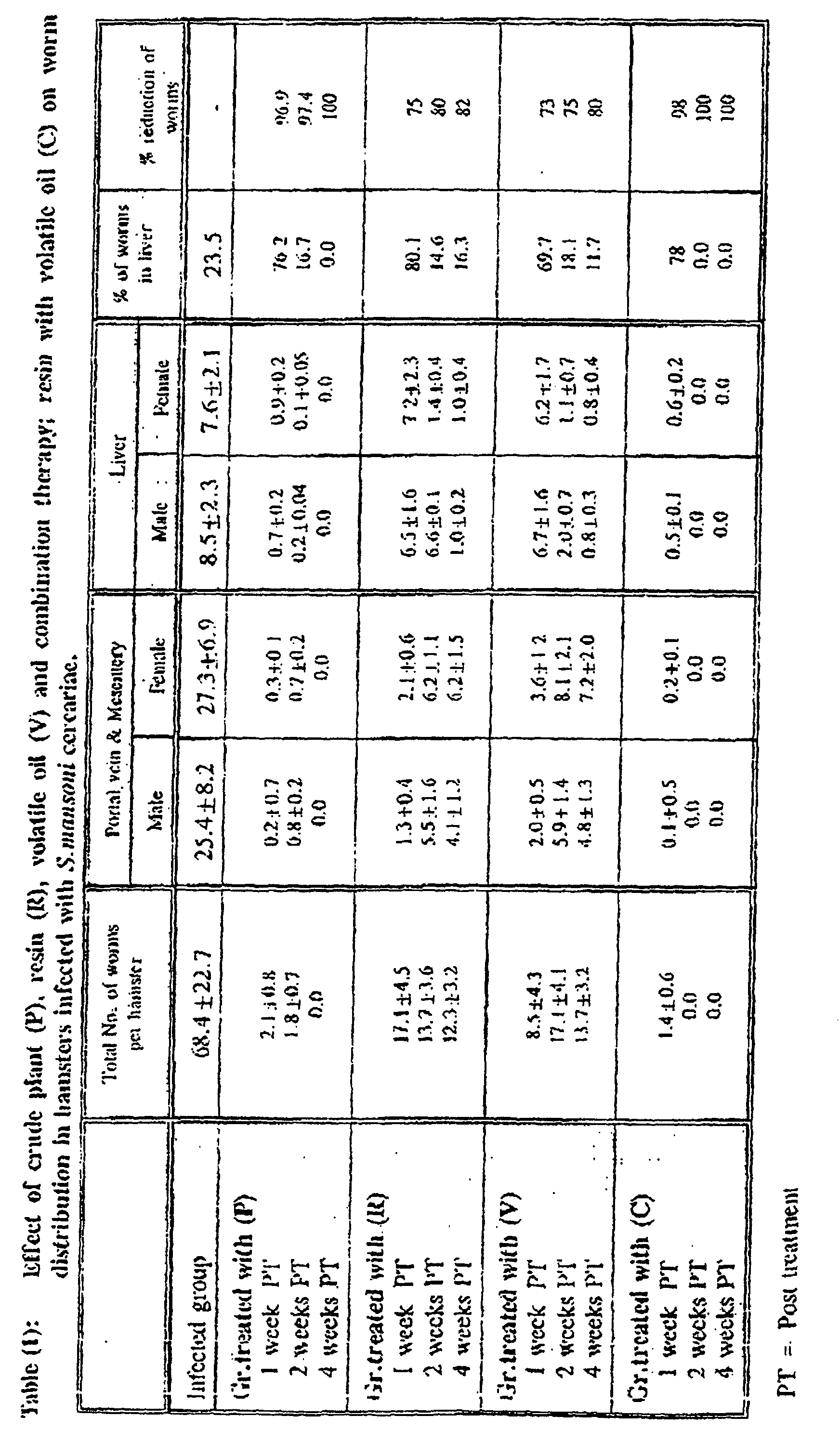

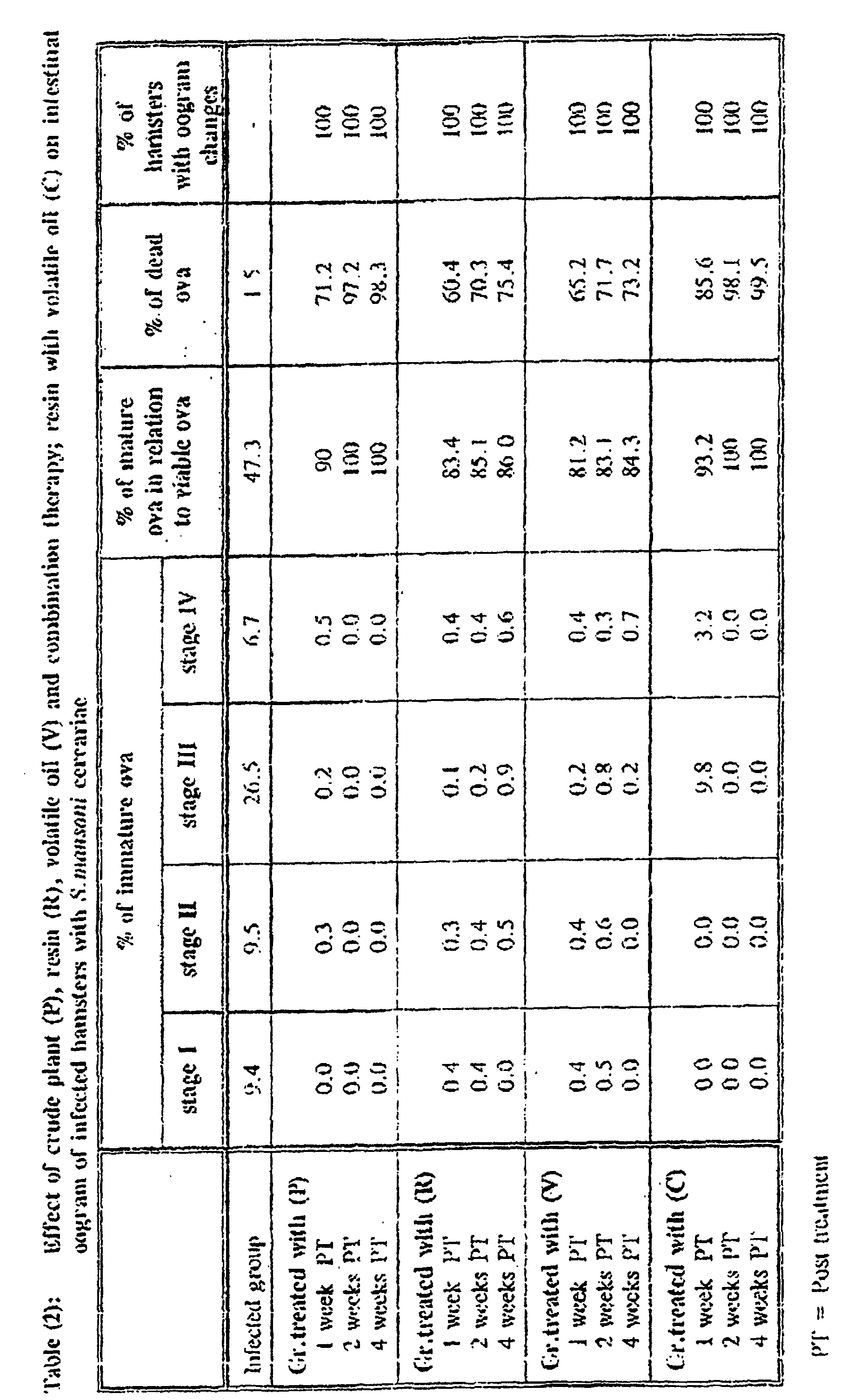

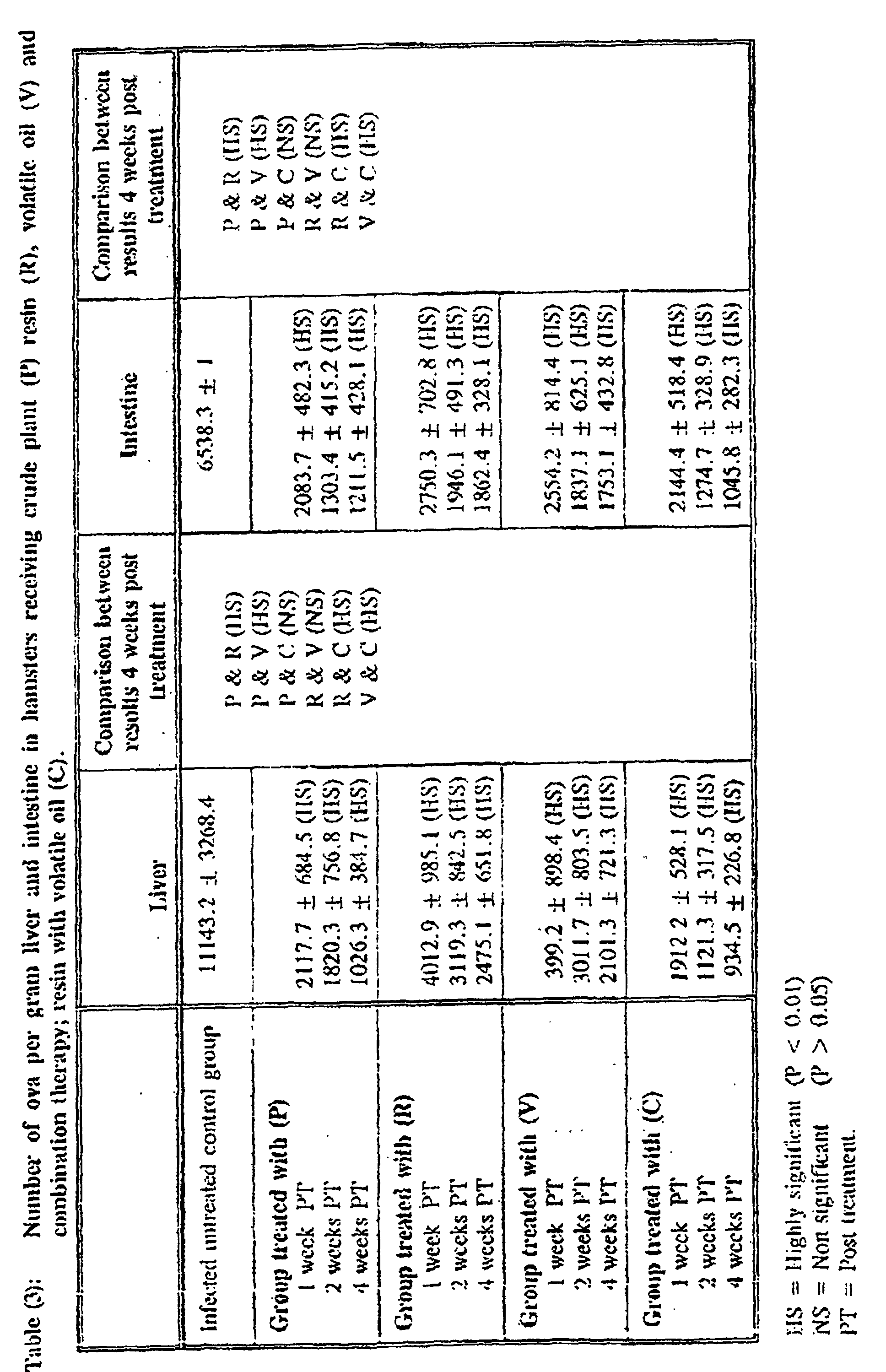

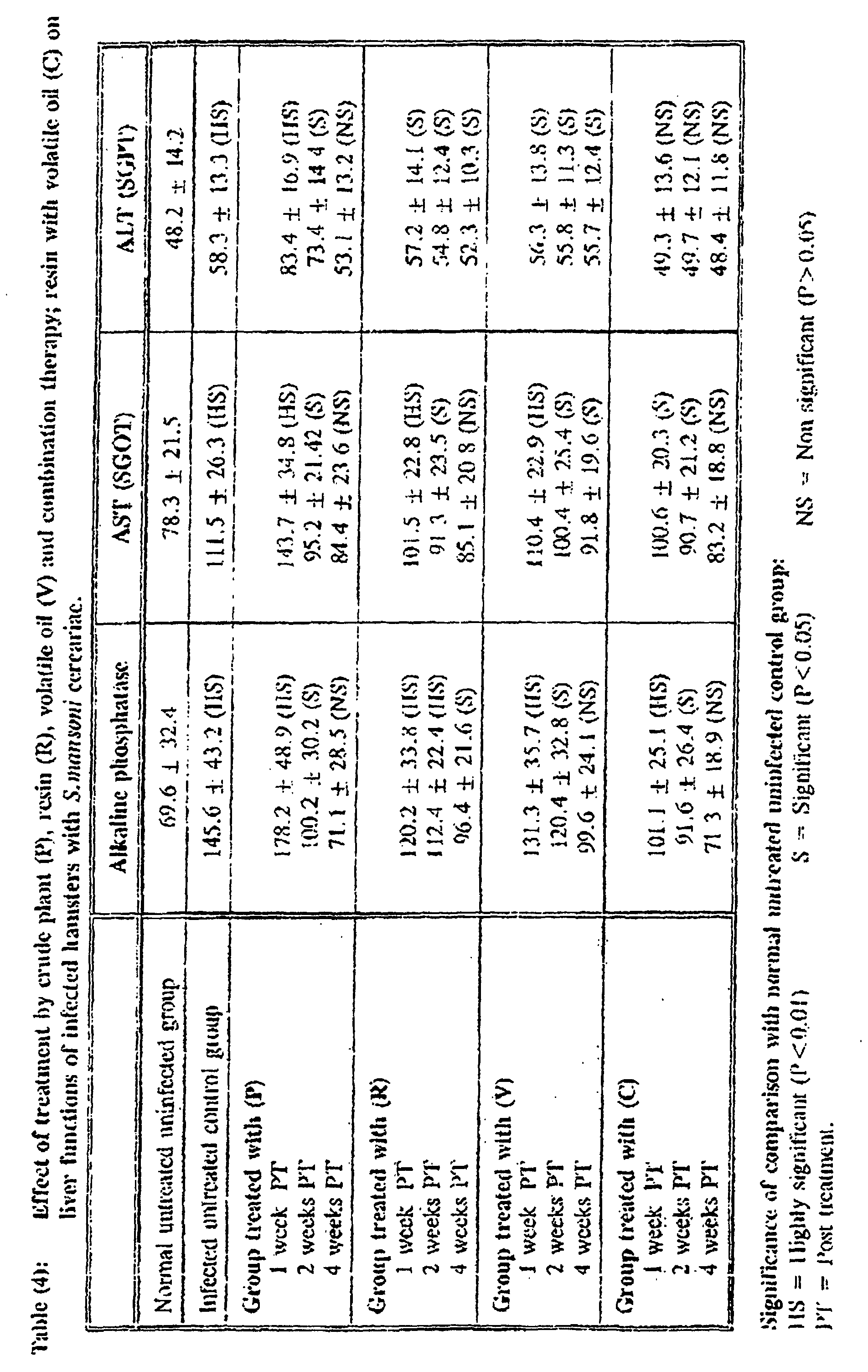

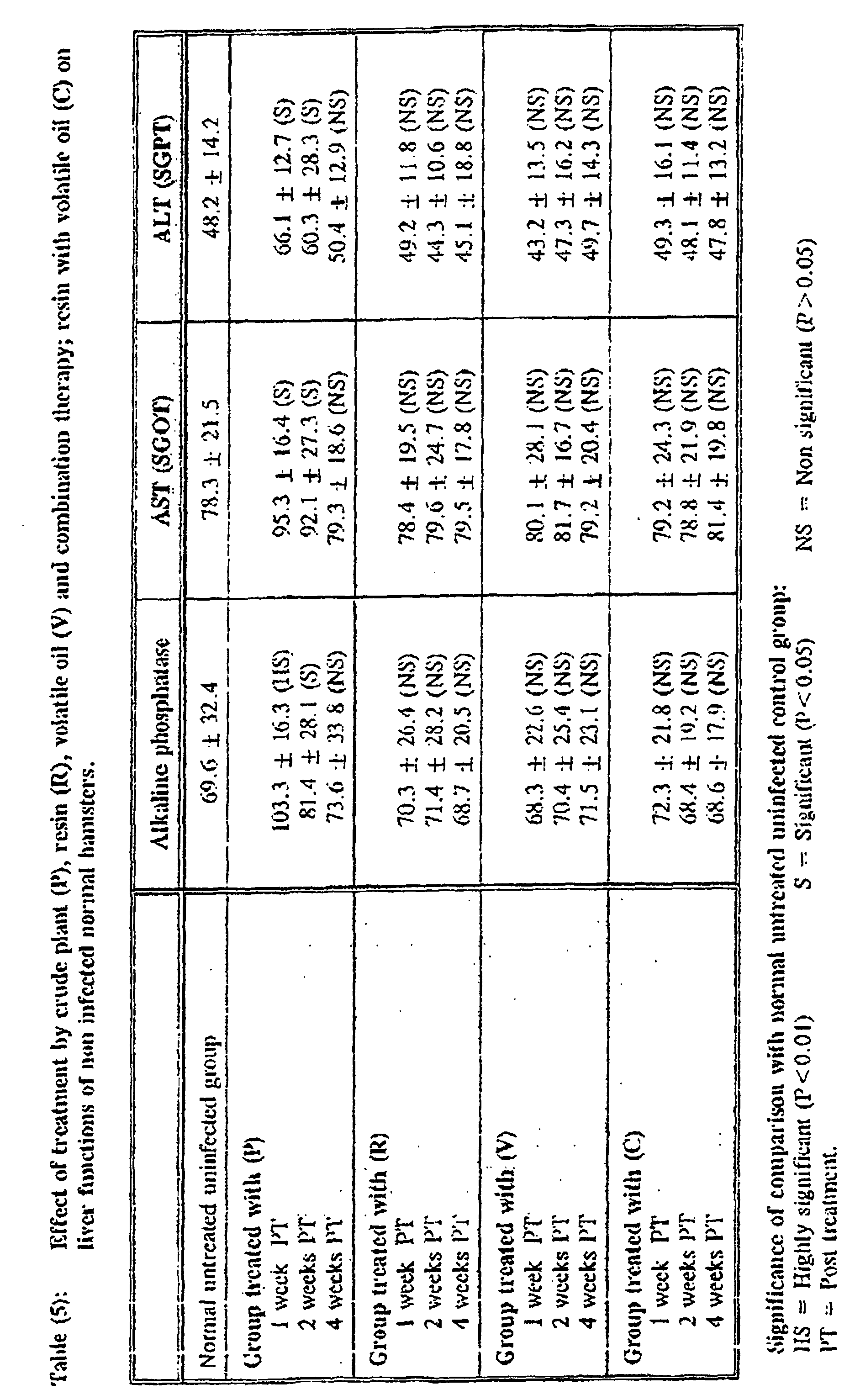

## Claims

1. Use of an extract from the Commiphora molmol plant comprising additionally or alternatively a volatile oil, resin or gum in the preparation of a medicament for the treatment of schistosomiasis.

2. Use according to claim 1, wherein said extract comprises oleo-gum resin (Myrrh).

3. A pharmaceutical composition comprising a pharmaceutically effective amount of an extract of Commiphora molmol comprising additionally or alternatively a volatile oil, resin or gum other than the oleo-gum resin, together with a pharmaceutically acceptable carrier diluent or excipient therefor.

4. A pharmaceutical composition according to claim 3 provided in, or in the form of, a gelatin capsule.

## Patentansprüche

1. Verwendung eines Extrakts der Commiphora molmol Pflanze umfassend zusätzlich oder alternativ ein flüchtiges Öl, Harz oder Gummi, bei der Herstellung eines Arzneimittels zur Behandlung von Schistosomiasis.

2. Verwendung nach Anspruch 1, wobei das Extrakt Öl-Gummi-Harz (Myrrhe) umfasst.

3. Pharmazeutische Zusammensetzung umfassend eine pharmazeutisch wirksame Menge eines Extrakts von Commiphora molmol umfassend zusätzlich oder alternativ ein von dem Öl-Gummi-Harz verschiedenes flüchtiges Öl, Harz oder Gummi, zusammen mit einem pharmazeutisch annehmbaren Träger, Verdünnungsmittel oder Hilfsmittel dafür.

4. Pharmazeutische Zusammensetzung nach Anspruch 3, bereitgestellt in oder in Form einer Gelatine-Kapsel.

## Revendications

1. Utilisation d'un extrait de la plante Commiphora molmol comprenant en outre ou en variante une huile volatile, résine ou gomme dans la préparation d'un médicament pour le traitement de la schistosomiase.

2. Utilisation suivant la revendication 1, dans laquelle l'extrait comprend une oléogomme-résine (myrrhe).

3. Composition pharmaceutique comprenant une quantité pharmaceutiquement efficace d'un extrait de Commiphora molmol comprenant en outre ou en variante une huile volatile, résine ou gomme autre que l'oléogomme-résine, conjointement avec un support, diluant ou excipient pharmaceutiquement acceptable.

4. Composition pharmaceutique suivant la revendication 3, fournie dans une, ou sous forme d'une, capsule de gélatine.
